# EUROPÄISCHE PATENTSCHRIFT

(11) **EP 4 225 397 B1**
(45) Veröffentlichungstag und Bekanntmachung des Hinweises auf die Patenterteilung: **11.03.2026**
(21) Anmeldenummer: 21789626.5
(22) Anmeldetag: 01.10.2021
(51) Int. Cl.: A61M 1/36

(54) **AUTOMATISCHES ENTLEEREN EINES DIALYSATORS NACH EINER BLUTBEHANDLUNGSTHERAPIE**
AUTOMATED EMPTYING OF A DIALYZER AFTER A BLOOD TREATMENT THERAPY
VIDANGE AUTOMATISÉE D'UN DIALYSEUR APRÈS UNE THÉRAPIE DE TRAITEMENT DU SANG

(30) Priorität: 07.10.2020 DE 102020126303
(43) Veröffentlichungstag der Anmeldung: 16.08.2023
(73) Patentinhaber: B. Braun Avitum AG, 34212 Melsungen (DE)
(72) Erfinder: SCHILLER, Joana Sophie, 48429 Rheine (DE); WÜRSCHMIDT, Tobias, 34346 Hann. Münden (DE)
(74) Vertreter: Winter, Brandl - Partnerschaft mbB
(86) Internationale Anmeldenummer: PCT/EP2021/077052
(87) Internationale Veröffentlichungsnummer: WO 2022/073857

(56) Entgegenhaltungen:
- EP-A1- 1 996 253

## Beschreibung

Die vorliegende Offenbarung betrifft eine extrakorporale Blutbehandlungsvorrichtung, insbesondere Dialysemaschine, zur Verwendung in einer Blutbehandlungstherapie, mit: einem extrakorporalen Kreislauf; einem Dialysierflüssigkeitskreislauf; und einem Dialysator umfassend eine Blutseite, welche mit dem extrakorporalen Kreislauf fluidisch verbunden ist, und eine Dialysierflüssigkeitsseite, welche mit dem Dialysierflüssigkeitskreislauf fluidisch verbunden ist, wobei die Blutseite des Dialysators und die Dialysierflüssigkeitsseite des Dialysators über eine in dem Dialysator vorgesehene Membran voneinander getrennt sind, und die extrakorporale Blutbehandlungsvorrichtung ferner eine Steuereinheit enthält, welche zum automatischen Entleeren des Dialysators nach einem Ende der Blutbehandlungstherapie durch Einstellen eines negativen Drucks bzw. Unterdrucks in dem Dialysierflüssigkeitskreislauf und einem damit einhergehenden Übertritt einer Flüssigkeit von der Blutseite über die Membran des Dialysators in die Dialysierflüssigkeitsseite eingerichtet ist. Ferner betrifft die vorliegende Offenbarung ein Verfahren zum automatischen Entleeren eines Dialysators nach einem Ende einer Blutbehandlungstherapie.

### Technischer Hintergrund

Nach einer erfolgten Blutbehandlungstherapie, in welcher Blut eines Patienten unter Verwendung eines Dialysators und gegebenenfalls einer den Dialysator durchströmenden Dialysierflüssigkeit extrakorporal gereinigt wurde, wird grundsätzlich das Blut des Patienten, welches sich noch in einem extrakorporalen Kreislauf, beispielsweise in einem arteriellen und venösen Schlauchsystem (A/V-Schlauchsystem), befindet, dem Patienten wiederzugeführt. Dieser Vorgang wird als Reinfusion bezeichnet. Erst wenn das in dem extrakorporalen Kreislauf noch vorhandene Blut dem Patienten in geeigneter Weise wiederzugeführt/ reinfundiert wurde, wird der Patient von dem extrakorporalen Schlauchsystem vollständig (sowohl arteriell als auch venös) abgekoppelt. Nach der Reinfusion befindet sich grundsätzlich noch (Reinfusions-)Flüssigkeit in dem extrakorporalen Kreislauf, dem Dialysator, etc. Vor einer Entsorgung der den extrakorporalen Kreislauf und den Dialysierflüssigkeitskreislauf bildenden Schläuche und des Dialysators sollten diese noch geleert/ entleert werden, insbesondere um die (gewichtsabhängigen) Entsorgungskosten zu reduzieren.

### Stand der Technik

Aus dem Stand der Technik sind zum einen extrakorporale Blutbehandlungsvorrichtungen bekannt, welche beim Entleeren eines Dialysators nach einem Ende einer Blutbehandlungstherapie und nach einer Reinfusion des Patientenblutes mehrere manuell durch einen Anwender durchzuführende Schritte erfordern. Beispielsweise ist es bekannt, dass zunächst der extrakorporale Kreislauf bzw. das Blutschlauchsystem von dem Dialysator abgekoppelt wird und in einen Beutel oder in einen Waste-Port der Blutbehandlungsvorrichtung geleert wird. Im Anschluss kann ein Dialysierflüssigkeitszuflussschlauch von dem Dialysator abgekoppelt werden und im Dialysator noch vorhandene Flüssigkeit zumindest zum Teil über den Dialysierflüssigkeitsabflussschlauch abgesaugt werden.

Diese teilautomatische Entleerung erfordert in nachteilhafter Weise mehrere manuell durchzuführende Schritte. Das Pflegepersonal muss mehrmals mit der extrakorporalen Blutbehandlungsvorrichtung interagieren, woraus sich insbesondere auch eine Wartezeit vor der Maschine ergibt. Weiterhin wird durch dieses Entleer-Verfahren zwar die Dialysierflüssigkeitsseite des Dialysators in geeigneter Weise von der noch darin vorhandenen Flüssigkeit befreit, nicht jedoch die Blutseite des Dialysators. Somit ist das Gewicht des Dialysators nach dem Entleeren noch sehr hoch, was mit hohen Entsorgungskosten einhergeht.

Aus der EP 3 231 466 B1 ist ein automatisiertes Entleeren eines extrakorporalen Kreislaufs/ AN-Schlauchsystems und einer Blutseite des Dialysators bekannt. Dabei wird auf der Dialysierflüssigkeitsseite des Dialysators ein Unterdruck erzeugt, mittels welchem eine Flüssigkeit auf der Blutseite des Dialysators abgesaugt wird, so dass die Flüssigkeit über eine Membran des Dialysators von der Blutseite zu der Dialysierflüssigkeitsseite übertritt. Der Flüssigkeitsübertritt wird dabei durch eine in dem extrakorporalen Kreislauf vorgesehene Pegel-/ oder Levelregulierungspumpe unterstützt. Während des Entleerungsvorgangs sind die Enden des arteriellen Abschnitts des extrakorporalen Kreislaufs und des venösen Abschnitts des extrakorporalen Kreislaufs kurzgeschlossen und ist zumindest eine in dem extrakorporalen Kreislauf vorgesehene Blutpumpe in Betrieb, so dass die im kurzgeschlossenen Blutkreislauf vorhandene Flüssigkeit in Therapiefließrichtung in die Blutseite des Dialysators gefördert wird, dort durch das Druckgefälle in die Dialysierflüssigkeitsseite des Dialysators übertritt und abgezogen wird.

Die EP 3 231 466 B1 beschäftigt sich nicht mit einem Entleeren der Dialysierflüssigkeitsseite des Dialysators. Vor diesem Hintergrund ist davon auszugehen, dass auch nach dem in der EP 3 231 466 B1 beschriebenen Entleeren der Blutseite des Dialysators und des extrakorporalen Kreislaufs mehrere manuell durchzuführende Schritte erforderlich sind (Abkoppeln des extrakorporalen Kreislaufs von dem Dialysator, Abkoppeln des Dialysierflüssigkeitszuflussschlauchs von dem Dialysator; Absaugen der noch in dem Dialysator vorhandenen Flüssigkeit über den Dialysierflüssigkeitsabflussschlauch). Daher kann zwar unter Heranziehung der Offenbarung der EP 3 231 466 B1 ein Gewicht des Dialysators nach dem Entleeren verringert werden, jedoch sind immer noch mehrere manuell durchzuführende Schritte von dem Anwender erforderlich.

Aus der EP 1 996 253 B1 ist schließlich bereits ein vollautomatisches Entleeren eines Dialysators nach einer Blutbehandlungstherapie bekannt. Gemäß der Offenbarung der EP 1 996 253 B1 wird zunächst die Blutseite des Dialysators und der extrakorporale Kreislauf in ähnlicher Weise wie in der EP 3 231 466 B1 entleert. Im Anschluss daran wird auch die Dialysierflüssigkeitsseite des Dialysators automatisch entleert. Dazu wird ein sich in dem Dialysierflüssigkeitszufluss befindliches (Entlüftungs-) Ventil geöffnet. Wenn nun eine sich in dem Dialysierflüssigkeitsabfluss befindliche Fluidpumpe betätigt wird, kann Luft in den Dialysator einströmen (gegebenenfalls unterstützt von einem sich in dem Dialysierflüssigkeitszufluss befindlichen Kompressor), so dass die sich noch auf der Dialysierflüssigkeitsseite des Dialysators befindliche Flüssigkeit hin zu dem Dialysatorausgang abtransportiert wird, bis der gesamte Dialysator mit Luft gefüllt ist.

In der EP 1 996 253 B1 wird in nachteilhafter Weise ein zusätzliches Entlüftungsventil benötigt, um das vollautomatische Entleeren des Dialysators bereitstellen zu können.

Sowohl EP 1 996 253 B1 als auch EP 3 231 466 B1 offenbaren eine extrakorporale Blutbehandlungsvorrichtung gemäß der Präambel des Anspruchs 1.

### Kurzbeschreibung der Offenbarung

Vor diesem Hintergrund ist es Aufgabe der vorliegenden Offenbarung die Nachteile des Standes der Technik zu vermeiden oder wenigstens zu mildern. Insbesondere soll ein Dialysator nach einem Ende einer Blutbehandlungstherapie vollständig automatisch geleert werden, um ein Entsorgungsgewicht des Dialysators auf ein Minimum zu reduzieren. Weiterhin soll eine Interaktion des Pflegepersonals mit der extrakorporalen Blutbehandlungsvorrichtung verringert werden. Darüber hinaus sollen für die automatische Entleerung bevorzugt lediglich die bereits standardmäßig in einer extrakorporalen Blutbehandlungsvorrichtung vorhandenen Komponenten verwendet werden/ erforderlich sein.

Diese Aufgabe wird durch eine extrakorporale Blutbehandlungsvorrichtung nach Anspruch 1 und ein Verfahren zum automatischen Entleeren eines Dialysators nach einem Ende einer Blutbehandlungstherapie nach Anspruch 12 gelöst. Vorteilhafte Weiterbildungen und Ausführungsformen sind in den Unteransprüchen beansprucht und/oder werden nachfolgend erläutert.

Die Offenbarung betrifft zunächst eine extrakorporale Blutbehandlungsvorrichtung zur Verwendung in einer Blutbehandlungstherapie, mit: einem extrakorporalen Kreislauf; einem Dialysierflüssigkeitskreislauf; und einem Dialysator umfassend eine Blutseite, welche mit dem extrakorporalen Kreislauf fluidisch verbunden ist, und eine Dialysierflüssigkeitsseite, welche mit dem Dialysierflüssigkeitskreislauf fluidisch verbunden ist, wobei die Blutseite des Dialysators und die Dialysierflüssigkeitsseite des Dialysators über eine in dem Dialysator vorgesehen Membran voneinander getrennt sind, und die extrakorporale Blutbehandlungsvorrichtung ferner eine Steuereinheit enthält, welche zum automatischen Entleeren des Dialysators nach einem Ende der Blutbehandlungstherapie durch Einstellen eines negativen Drucks bzw. Unterdrucks in dem Dialysierflüssigkeitskreislauf und einem damit einhergehenden Übertritt einer Flüssigkeit von der Blutseite über die Membran des Dialysators in die Dialysierflüssigkeitsseite eingerichtet ist. Die Steuereinheit ist eingerichtet, wenn die Flüssigkeit aus dem extrakorporalen Kreislauf vollständig von der Blutseite über die Membran des Dialysators in die Dialysierflüssigkeitsseite übergetreten ist und die Blutseite des Dialysators geleert ist, durch ein fortgesetztes Einstellen des negativen Drucks bzw. Unterdrucks in dem Dialysierflüssigkeitskreislauf einen Übertritt von Luft von der Blutseite über die Membran des Dialysators zu der Dialysierflüssigkeitsseite und ein Verdrängen der Flüssigkeit aus der Dialysierflüssigkeitsseite des Dialysators heraus hin zu einem Dialysierflüssigkeitsabfluss (stromabwärts des Dialysators) zu bewirken, um auch die Dialysierflüssigkeitsseite des Dialysators automatisch zu entleeren.

Die Steuereinheit steuert gemäß der vorliegenden Offenbarung nach einem Ende der Blutbehandlungstherapie bevorzugt zunächst eine Reinfusion von Blut in den Patienten derart, dass eine (Dialysier-) Flüssigkeit von dem Dialysierflüssigkeitskreislauf über die Membran des Dialysators an den extrakorporalen Kreislauf geliefert wird, welche bei der Reinfusion das in dem extrakorporalen Kreislauf vorhandene Blut zu einem Patienten hin verdrängt, um das Blut dem Patienten sowohl über den venösen Abschnitt als auch über den arteriellen Abschnitt zurückzugeben.

Bevorzugt ist die Steuereinheit eingerichtet, wenn die Reinfusion (sowohl über den arteriellen Abschnitt des extrakorporalen Kreislaufs als auch über den venösen Abschnitt des extrakorporalen Kreislaufs) abgebrochen wurde, eine in dem arteriellen Abschnitt des extrakorporalen Kreislaufs vorgesehene arterielle Schlauchklemme sowie eine in dem venösen Abschnitt des extrakorporalen Kreislaufs vorgesehene venöse Schlauchklemme zu schließen.

In vorteilhafter Weise wird im Anschluss der Patient von einem Pflegepersonal sowohl arteriell als auch venös abgekoppelt.

Nach dem Abkoppeln des Patienten werden bevorzugt ein Ende/ patientenseitiger Anschluss des arteriellen Abschnitts mit einem Ende/ patientenseitigen Anschluss des venösen Abschnitts kurgeschlossen bzw. miteinander verbunden. Dies stellt in vorteilhafter Weise die letzte aktive Interaktion des Pflegepersonals mit der extrakorporalen Blutbehandlungsvorrichtung dar. In anderen Worten ist es offenbarungsgemäß vorgesehen, dass im Anschluss eine von der Steuereinheit gesteuerte vollständige automatische Entleerung des Dialysators durchgeführt wird, ohne dass das Pflegepersonal noch einmal mit der extrakorporalen Blutbehandlungsvorrichtung interagieren muss.

Es ist ferner zweckmäßig, wenn der Dialysator beim automatischen Entleeren desselben derart an der extrakorporalen Blutbehandlungsvorrichtung angeordnet bzw. ausgerichtet ist, dass sich ein Dialysierflüssigkeitsausgang, welcher mit dem Dialysierflüssigkeitsabfluss verbunden ist, (in einer Höhenrichtung/ Vertikalrichtung/ Hochrichtung der extrakorporalen Blutbehandlungsvorrichtung) unter einem Dialysierflüssigkeitseingang, welcher mit einem Diaylsierflüssigkeitszufluss verbunden ist, angeordnet ist.

In anderen Worten ist der Dialysator beim automatischen Entleeren desselben bevorzugt derart an der extrakorporalen Blutbehandlungsvorrichtung angeordnet/ ausgerichtet, dass sich ein Dialysierflüssigkeitsausgang unten und ein Dialysierflüssigkeitseingang oben befindet. Besonders bevorzugt ist der Dialysator dabei vertikal ausgerichtet, das heißt, eine Längsachse des im Wesentlichen zylinderförmigen Dialysators erstreckt sich bevorzugt in einer vertikalen Richtung (senkrecht auf die Erdoberfläche).

Wenn bei einer extrakorporalen Blutbehandlungsvorrichtung grundsätzlich vorgesehen ist, dass sich der Dialysatorausgang während der Blutbehandlungstherapie oben und nicht unten befindet (beispielsweise bedingt durch das Gegenstromprinzip), ist es erforderlich, dass das Pflegepersonal den Dialysator vor der automatischen Entleerung dreht, das heißt so ausrichtet, dass sich der Dialysierflüssigkeitsausgang unten befindet. Dieser gegebenenfalls vorzunehmende manuelle Schritt kann von dem Pflegepersonal zusammen mit dem Kurzschließen des extrakorporalen Kreislaufs vorgenommen werden, so dass bevorzugt keine zusätzliche, mit einer Wartezeit des Pflegepersonals verbundene Interaktion erforderlich wird.

In vorteilhafter Weise wird im Anschluss an das Abkoppeln des Patienten und an das möglicherweise vorzunehmende Drehen des Dialysators zunächst ein automatisches Entleeren der Blutseite des Dialysators vorgenommen. Insbesondere kann dabei das in der EP 3 231 466 B1 beschriebene Verfahren durchgeführt werden, welches ein geeignetes Verfahren zum Leeren des Blutschlauchsystems und des blutseitigen Dialysators/ der Blutseite des Dialysators bereitstellt. Insbesondere ist die Steuereinheit somit eingerichtet, einen negativen Druck bzw. Unterdruck in dem Dialysierflüssigkeitskreislauf (auf der Dialysierflüssigkeitsseite) aufzubauen/ zu erzeugen, wodurch eine in dem extrakorporalen Kreislauf (auf der Blutseite) vorhandene (Dialysier-) Flüssigkeit abgesaugt wird. Dabei wird die (in dem extrakorporalen Kreislauf noch vorhandene) Flüssigkeit beim Entleeren bevorzugt von dem extrakorporalen Kreislauf über die Membran des Dialysators an den Dialysierflüssigkeitskreislauf geliefert, und zwar solange, bis keine Flüssigkeit mehr in dem extrakorporalen Kreislauf und der Blutseite des Dialysators vorhanden ist.

Kern der Offenbarung ist es, dass die Steuereinheit eingerichtet ist, das Entleeren des Dialysators derart zu steuern, dass zunächst die Flüssigkeit und anschließend (wenn die Flüssigkeit aus dem extrakorporalen Kreislauf vollständig über die Membran des Dialysators von der Blutseite zu der Dialysierflüssigkeitsseite übergetreten ist, d.h. sich keine Flüssigkeit mehr in dem extrakorporalen Kreislauf und der Blutseite des Dialysators befindet) Luft von dem extrakorporalen Kreislauf/ der Blutseite über die Membran des Dialysators zum Dialysierflüssigkeitskreislauf/ der Dialysierflüssigkeitsseite übertritt, so dass die Dialysierflüssigkeit durch die übertretende Luft auch aus der Dialysierflüssigkeitsseite des Dialysators heraus hin zum Dialysierflüssigkeitsabfluss verdrängt wird.

Insbesondere hat sich gemäß der vorliegenden Offenbarung herausgestellt, dass, wenn die Flüssigkeit aus dem extrakorporalen Kreislauf vollständig von der Blutseite über die Membran des Dialysators in die Dialysierflüssigkeitsseite übergetreten ist und die Blutseite des Dialysators geleert ist, durch ein fortgesetztes Einstellen des negativen Drucks bzw. Unterdrucks in dem Dialysierflüssigkeitskreislauf (Saugdruck auf der Dialysierflüssigkeitsseite) erreicht werden kann, dass Luft langsam von der Blutseite über die Membran des Dialysators zu der Dialysierflüssigkeitsseite übertritt. Wenn nun der Dialysator so ausgerichtet ist, dass sich der Dialysatorausgang bzw. der Dialysierflüssigkeitsabfluss unten und der Dialysatoreingang bzw. Dialysierflüssigkeitszufluss oben befindet, sammelt sich die übertretende Luft in einem oberen Abschnitt des Dialysators (nahe dem Dialysatorseingang), verdrängt die Flüssigkeit nach unten hin zum Dialysatorausgang und drückt die in der Dialysierflüssigkeitsseite noch vorhandene Flüssigkeit über den Dialysatorausgang aus dem Dialysator heraus.

Die Steuereinheit ist somit insgesamt zum vollständigen automatischen Entleeren des Dialysators nach einem Ende der Blutbehandlungstherapie eingerichtet.

Bevorzugt ist die Steuereinheit eingerichtet, den negativen Druck bzw. Unterdruck in dem Dialysierflüssigkeitskreislauf derart zu erzeugen, dass eine Flusspumpe-Ausgang, welche eine Fluidpumpe in dem Dialysierflüssigkeitsabfluss des Dialysierflüssigkeitskreislaufs (stromabwärts des Dialysators) ist, angesteuert wird, die Flüssigkeit oder die Luft (von der Blutseite über die Membran des Dialysators in die Dialysierflüssigkeitsseite und schließlich) aus dem Dialysator heraus/ von dem Dialysator weg in den Dialysierflüssigkeitsabfluss zu pumpen.

In vorteilhafter Weise ist die Steuereinheit dabei eingerichtet, eine in dem extrakorporalen Kreislauf vorgesehene Kompressorpumpe anzusteuern/ zu betätigen, damit diese den Übertritt der Flüssigkeit und/ oder der Luft von der Blutseite über die Membran des Dialysators hin zu der Dialysierflüssigkeitsseite unterstützt. Insbesondere ist die Steuereinheit eingerichtet, die Kompressorpumpe derart anzusteuern, dass diese die Flüssigkeit und die Luft über die Membran des Dialysators drückt.

In vorteilhafter Weise ist die Steuereinheit weiterhin eingerichtet, beim automatischen Entleeren des Dialysators einen Transmembrandruck des Dialysators auf einen Druck zu steuern oder zu regeln, welcher größer einem vorbestimmten Wert und kleiner einem dialysatorspezifischen, maximal zulässigen Transmembrandruck ist. Der maximal zulässige Transmembrandruck ist insbesondere vom Dialysatortyp/ vom verwendeten Dialysator abhängig und ist grundsätzlich in Datenblättern von Dialysatoren mit angegeben.

Bevorzugt ist die Steuereinheit dabei eingerichtet, den Transmembrandruck des Dialysators auf einen Druck zu steuern oder zu regeln, welcher größer als 400 mmHg, bevorzugt größer als 500 mmHg ist. Beispielsweise wird der Transmembrandruck von der Steuereinheit auf einen Druck zwischen 500 mmHg und 600 mmHg, beispielsweise 550 mmHg, gesteuert/ geregelt, aber nur, wenn bei dem eingestellten Druck der maximal zulässige Transmembrandruck noch nicht überschritten wird. Es muss unbedingt darauf geachtet werden, dass der maximal zulässige Transmembrandruck nicht überschritten wird. Bevorzugt können in der extrakorporalen Blutbehandlungsvorrichtung der vorliegenden Offenbarung auch lediglich Dialysatoren eingesetzt werden, deren maximal zulässiger Transmembrandruck zumindest größer als 600 mmHg ist.

Es hat sich gemäß der vorliegenden Offenbarung insbesondere herausgestellt, dass, wenn ein Unterdruck auf der Dialysierflüssigkeitsseite (durch Ansteuern der Flusspumpe-Ausgang) gegebenenfalls zusammen mit einem Überdruck auf der Blutseite (durch Ansteuern der Kompressorpumpe) derart erzeugt wird, dass ein konstanter und hoher Transmembrandruck vorliegt, welcher in dem voranstehend beschriebenen Bereich liegt, bei einem bereits entleerten bzw. geleerten extrakorporalen Kreislauf Luft (relativ langsam) durch die Membran des Dialysators tritt und die auf der Dialysierflüssigkeitsseite des Dialysators noch vorhandene Dialysierflüssigkeit auch aus dem Dialysator verdrängt.

Bevorzugt ist die Steuereinheit eingerichtet, das automatische Entleeren der Dialysierflüssigkeitsseite des Dialysators sensorgesteuert/ -geregelt abzubrechen.

Gemäß einem bevorzugten Ausführungsbeispiel ist die Steuereinheit eingerichtet, ein Drucksignal bzw. einen Druckverlauf eines in dem Dialysierflüssigkeitsabfluss angeordneten Drucksensors, welcher einen Druck in dem Dialysierflüssigkeitsabfluss misst bzw. überwacht, auszuwerten, und das automatische Entleeren der Diaylsierflüssigkeitsseite auf der Grundlage des Drucksignals bzw. des Druckverlaufs des Drucksensors abzubrechen.

Dabei ist die Steuereinheit bevorzugt eingerichtet, eine Steigung bzw. eine erste Ableitung des Drucksignals bzw. des Druckverlaufs des Drucksensors auszuwerten, und das automatische Entleeren der Dialysierflüssigkeitsseite abzubrechen, wenn die Steigung bzw. erste Ableitung des Drucksignals bzw. des Druckverlaufs einen vorbestimmten ersten Grenzwert unterschreitet. Insbesondere hat sich offenbarungsgemäß herausgestellt, dass sich Luft in dem Dialysierflüssigkeitsabfluss befindet, wenn die Steigung/ die erste Ableitung des Drucksignals bzw. des Druckverlaufs des Drucksensors negativ wird.

Gemäß einem alternativen bevorzugten Ausführungsbeispiel kann die Steuereinheit auch eingerichtet sein, das automatische Entleeren der Dialysierflüssigkeitsseite sensorgesteuert abzubrechen, wenn von einem in dem Dialysierflüssigkeitskreislauf vorgesehenen bzw. angeordneten Luftabscheider erfasst bzw. gemessen wird, dass ein Flüssigkeitspegel des Luftabscheiders unter einen vorbestimmten Flüssigkeitspegel bzw. Pegelstand gefallen ist.

Alternativ oder zusätzlich kann die Steuereinheit auch eingerichtet sein, das automatische Entleeren der Dialysierflüssigkeitsseite des Dialysators zeitgesteuert/ - geregelt abzubrechen. Insbesondere ist grundsätzlich auch eine Steuerung denkbar, welche einen sensorgesteuerten Abbruch mit einem zeitgesteuerten Abbruch kombiniert.

Weiterhin betrifft die vorliegende Offenbarung ein Verfahren zum automatischen Entleeren eines Dialysators nach einem Ende einer Blutbehandlungstherapie, insbesondere durchgeführt oder durchzuführen in einer extrakorporalen Blutbehandlungsvorrichtung wie voranstehend beschrieben, mit den Schritten: Einstellen eines negativen Drucks bzw. Unterdrucks in einem Dialysierflüssigkeitskreislauf und damit einhergehendes Übertreten einer Flüssigkeit von einer Blutseite des Dialysators über eine Membran des Dialysators in eine Dialysierflüssigkeitsseite des Dialysators; und, wenn die Flüssigkeit aus einem extrakorporalen Kreislauf vollständig von der Blutseite über die Membran des Dialysators in die Dialysierflüssigkeitsseite übergetreten ist und die Blutseite des Dialysators geleert ist, fortgesetztes Einstellen des negativen Drucks bzw. Unterdrucks in dem Dialysierflüssigkeitskreislauf zum Bewirken eines Übertritts von Luft von der Blutseite über die Membran des Dialysators zu der Dialysierflüssigkeitsseite und eines Verdrängens der Flüssigkeit aus der Dialysierflüssigkeitsseite des Dialysators heraus hin zu einem Dialysierflüssigkeitsabfluss, um auch die Dialysierflüssigkeitsseite des Dialysators automatisch zu entleeren.

Bevorzugt weist das Verfahren ferner den folgenden Schritt auf: Anordnen oder Ausrichten des Dialysators beim automatischen Entleeren desselben derart an der extrakorporalen Blutbehandlungsvorrichtung, dass ein Dialysierflüssigkeitsausgang, welcher mit dem Dialysierflüssigkeitsabfluss verbunden ist, (in einer Höhenrichtung/ Vertikalrichtung/ Hochrichtung der extrakorporalen Blutbehandlungsvorrichtung) unter einem Dialysierflüssigkeitseingang, welcher mit einem Diaylsierflüssigkeitszufluss verbunden ist, angeordnet ist.

Es ist vorteilhaft, wenn das Verfahren außerdem den folgenden Schritt enthält: Erzeugen des negativen Drucks bzw. Unterdrucks in dem Dialysierflüssigkeitskreislauf durch ein Pumpen der Flüssigkeit oder der Luft aus dem Dialysator heraus in den Dialysierflüssigkeitsabfluss mittels einer Flusspumpe-Ausgang, welche eine Fluidpumpe in dem Dialysierflüssigkeitsabfluss des Dialysierflüssigkeitskreislaufs ist.

Bevorzugt weist das Verfahren weiterhin den folgenden Schritt auf: Unterstützen des Übertritts der Flüssigkeit oder der Luft von der Blutseite über die Membran des Dialysators hin zu der Dialysierflüssigkeitsseite durch eine in dem extrakorporalen Kreislauf vorgesehene Kompressorpumpe.

Insbesondere enthält das Verfahren außerdem folgenden Schritt: Steuern oder Regeln eines Transmembrandrucks des Dialysators auf einen Druck, welcher größer einem vorbestimmten Wert und kleiner einem dialysatorspezifischen, maximal zulässigen Transmembrandruck ist. Bevorzugt weist das Verfahren zusätzlich den Schritt auf:
Steuern oder Regeln des Transmembrandrucks des Dialysators auf einen Druck, welcher größer als 400 mmHg, bevorzugt größer als 500 mmHg ist.

Es ist von Vorteil, wenn das Verfahren weiterhin den folgenden Schritt enthält: Sensorgesteuertes Abbrechen des automatischen Entleerens der Dialysierflüssigkeitsseite des Dialysators.

Bevorzugt enthält das Verfahren ferner die folgenden Schritte: Auswerten eines Drucksignals bzw. eines Druckverlaufs eines in dem Dialysierflüssigkeitsabfluss angeordneten Drucksensors, welcher einen Druck in dem Dialysierflüssigkeitsabfluss misst bzw. überwacht, und Abbrechen des automatischen Entleerens der Diaylsierflüssigkeitsseite auf der Grundlage des Drucksignals bzw. des Druckverlaufs des Drucksensors.

Weiter bevorzugt enthält das Verfahren die Schritte: Auswerten einer Steigung bzw. einer ersten Ableitung des Drucksignals bzw. des Druckverlaufs des Drucksensors, und Abbrechen des automatischen Entleerens der Dialysierflüssigkeitsseite, wenn die Steigung bzw. erste Ableitung des Drucksignals bzw. des Druckverlaufs einen vorbestimmten ersten Grenzwert unterschreitet.

Alternativ weist das Verfahren bevorzugt den Schritt auf: Sensorgesteuertes Abbrechen des automatischen Entleerens der Dialysierflüssigkeitsseite, wenn von einem in dem Dialysierflüssigkeitskreislauf vorgesehenen bzw. angeordneten Luftabscheider erfasst bzw. gemessen wird, dass ein Flüssigkeitspegel des Luftabscheiders unter einen vorbestimmten Flüssigkeitspegel bzw. Pegelstand gefallen ist.

Alternativ oder zusätzlich enthält das Verfahren vorzugsweise den folgenden Schritt: Zeitgesteuertes Abbrechen des automatischen Entleerens der Dialysierflüssigkeitsseite des Dialysators.

Wenn das offenbarungsgemäße Verfahren durchgeführt wird, ist bevorzugt ein Ende des arteriellen Abschnitts des extrakorporalen Kreislaufs mit einem Ende des venösen Abschnitts des extrakorporalen Kreislaufs verbunden, so dass ein Patient bereits abgekoppelt ist. Das offenbarungsgemäße Verfahren betrifft somit kein Verfahren zur chirurgischen oder therapeutischen Behandlung des menschlichen Körpers.

### Kurzbeschreibung der Figuren

Die vorliegende Offenbarung wird nachfolgend anhand von Figuren weiter erläutert. Es zeigen:
- Fig. 1: eine extrakorporale Blutbehandlungsvorrichtung gemäß der vorliegenden Offenbarung während einer Blutbehandlungstherapie bzw. während einer Reinfusion;
- Fig. 2: die extrakorporale Blutbehandlungsvorrichtung gemäß der vorliegenden Offenbarung während eines automatischen Entleerens eines Dialysators;
- Fig. 3: eine Detailansicht des Dialysators in einem Zustand, in welchem eine Blutseite des Dialysators bereits vollständig entleert ist;
- Fig. 4: eine Detailansicht des Dialysators in einem Zustand, in welchem eine Dialysierflüssigkeitsseite des Dialysators gerade entleert wird;
- Fig. 5: ein Diagramm, in welchem ein Drucksignal bzw. ein Druckverlauf eines in dem Dialysierflüssigkeitsabfluss angeordneten Drucksensors über die Zeit dargestellt ist;
- Fig. 6: ein Diagramm, in welchem eine erste Ableitung des Drucksignals bzw. des Druckverlaufs des Drucksensors über die Zeit dargestellt ist; und
- Fig. 7: eine Tabelle, in welcher für verschiedene Dialysator-Entleerungsverfahren eine erforderliche Zeit und ein verbleibendes Entsorgungsgewicht angegeben ist.

### Figurenbeschreibung

Die Figuren sind lediglich schematischer Natur und dienen ausschließlich dem Verständnis der Offenbarung. Gleiche Elemente sind dabei mit denselben Bezugszeichen versehen. Die Merkmale der einzelnen Ausführungsbeispiele können untereinander ausgetauscht werden, wenn dies nicht explizit anders beschrieben wird.

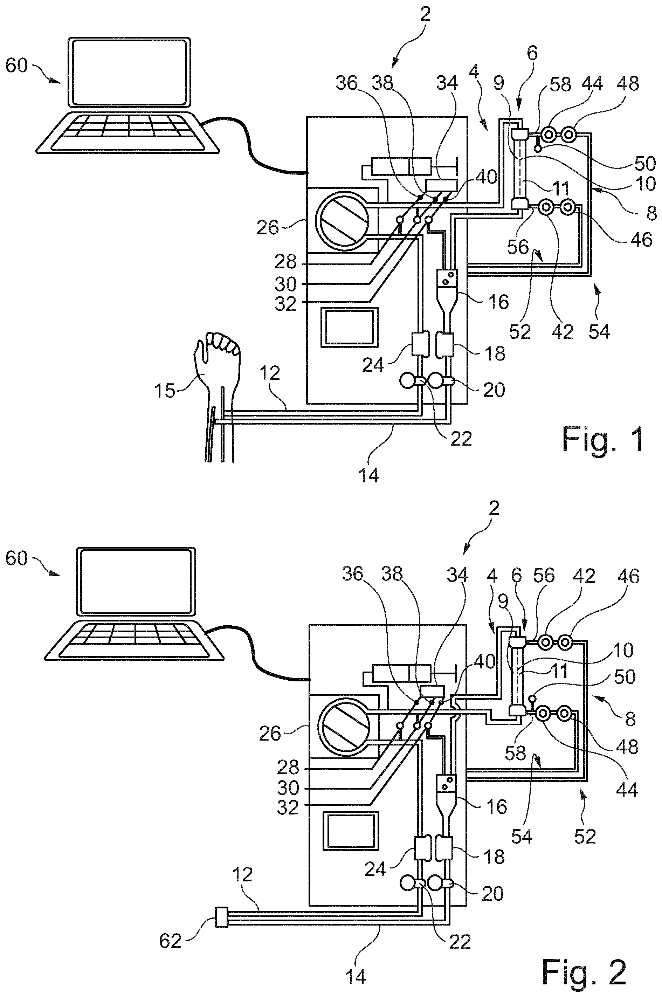

Fig. 1 zeigt eine extrakorporale Blutbehandlungsvorrichtung (Dialysemaschine) 2 während einer Blutbehandlungstherapie bzw. während einer Reinfusion, also vor dem offenbarungsgemäßen automatischen Entleeren.

Die extrakorporale Blutbehandlungsvorrichtung 2 enthält grundsätzlich einen extrakorporalen Kreislauf (A/V-Schlauchsystem) 4, einen Dialysator 6 und einen Dialysierflüssigkeitskreislauf 8. Eine Blutseite 9 des Dialysators 6 ist dabei über eine (Hohlfaser-) Membran 10 von einer Dialysierflüssigkeitsseite 11 des Dialysators 6 getrennt.

Der extrakorporale Kreislauf 4 enthält einen arteriellen Abschnitt 12, welcher sich stromaufwärts des Dialysators 6 befindet, und einen venösen Abschnitt 14, welcher sich stromabwärts des Dialysators 6 befindet.

Wie aus Fig. 1 ersichtlich ist, sind der arterielle Abschnitt 12 und der venöse Abschnitt 14 an einem Patienten 15 angekoppelt. In anderen Worten ist ein Ende des arteriellen Abschnitts 12 an eine Arterie des Patienten 15 angekoppelt und ist ein Ende des venösen Abschnitts 14 an eine Vene des Patienten 15 angekoppelt.

In dem venösen Abschnitt 14 des extrakorporalen Kreislaufs 4 sind stromabwärts des Dialysators 6 (das heißt ausgehend von dem Dialysator 6 in einer Richtung hin zu dem Ende des venösen Abschnitts 14) eine venöse Expansionskammer bzw. Luftfalle 16, ein venöser Sicherheitsluftdetektor 18 und eine venöse Schlauchklemme 20 vorgesehen.

In dem arteriellen Abschnitt 12 sind ausgehend von dem patientenseitigen Ende des arteriellen Abschnitts 12 in einer Richtung hin zu dem Dialysator 6 eine arterielle Schlauchklemme 22, ein arterieller Sicherheitsluftdetektor 24 und eine (arterielle) Blutpumpe 26 vorgesehen. Wie in Fig. 1 ersichtlich ist, ist der extrakorporale Kreislauf 4 (insbesondere ein Blutpumpenadapter desselben) bereits in die Blutpumpe 26, welche bevorzugt als Rollenpumpe bzw. Peristaltikpumpe ausgebildet ist und eingerichtet ist, durch Quetschen eines Schlauchs ein Fluid/ eine Flüssigkeit zu fördern, eingesetzt.

In dem arteriellen Abschnitt 12 kann ein arterieller Druck stromaufwärts bzw. vor der Blutpumpe 26 mit einem arteriellen Drucksensor 28 gemessen werden. Weiterhin kann ein Dialysatoreingangsdruck stromabwärts bzw. nach der Blutpumpe 26 und stromaufwärts bzw. vor dem Dialysator 6 (zwischen Dialysator 6 und Blutpumpe 26) über einen Dialysatoreingangsdrucksensor 30 gemessen werden. In dem venösen Abschnitt 14 kann ein venöser Druck an/ hinter der venösen Expansionskammer bzw. Luftfalle 16 über einen venösen Drucksensor 32 gemessen werden. Die in dem extrakorporalen Kreislauf 4 vorgesehenen Drucksensoren 28, 30, 32 können den Druck an den entsprechenden Stellen in dem extrakorporalen Kreislauf 4, an welchen diese angeordnet/ vorgesehen sind, messen/ abnehmen/ überwachen.

Wie aus Fig. 1 weiterhin hervorgeht, befindet sich hinter dem arteriellen Drucksensor 28, dem Dialysatoreingangsdrucksensor 30 und dem venösen Drucksensor 32 eine Kompressorpumpe bzw. eine Pegel- oder Levelregulierungspumpe (LRP) 34 mit dazugehörigen Ventilen 36, 38, 40, nämlich einem ersten Ventil 36 zwischen dem arteriellen Drucksensor 28 und der Kompressorpumpe 34, einem zweiten Ventil 38 zwischen dem Dialysatoreingangsdrucksensor 30 und der Kompressorpumpe 34, und einem dritten Ventil 40 zwischen dem venösen Drucksensor 32 und der Kompressorpumpe 34.

Der Dialysierflüssigkeitskreislauf 8 enthält ein Dialysatoreinlassventil 42, ein Dialysatorauslassventil 44, eine Flusspumpe-Eingang 46, eine Flusspumpe-Ausgang 48 und einen Drucksensor 50. Das Dialysatoreinlassventil 42 und die Flusspumpe-Eingang 46 sind dabei an einem Dialysierflüssigkeitszufluss 52 stromaufwärts des Dialysators 6 vorgesehen/ angeordnet. Der Drucksensor 50, das Dialysatorauslassventil 44 und die Flusspumpe-Ausgang 48 sind an einem Dialysierflüssigkeitsabfluss 54 stromabwärts des Dialysators 6 vorgesehen/ angeordnet. Die Flusspumpe-Eingang 46 und die Flusspumpe-Ausgang 48 sind bevorzugt Zahnradpumpen. Der Dialysierflüssigkeitszufluss 52 ist an einem Dialysatoreingang 56 des Dialysators 6 angekuppelt. Der Dialysierflüssigkeitsabfluss 54 ist an einem Dialysatorausgang 58 des Dialysators 6 angekuppelt.

Die extrakorporale Blutbehandlungsvorrichtung 2 weist weiterhin eine Steuereinheit 60 auf, welche bevorzugt als ein Prozessor, insbesondere als eine zentrale Rechen-/ bzw. Verarbeitungseinheit (CPU), ausgebildet ist. Die Steuereinheit 60 ist bevorzugt in die extrakorporale Blutbehandlungsvorrichtung 2 integriert, das heißt nicht von der extrakorporalen Blutbehandlungsvorrichtung 2 separiert. Die Steuereinheit 60 erhält Informationen von Sensoren, welche in der extrakorporalen Blutbehandlungsvorrichtung 2 vorgesehen sind. Lediglich beispielhaft sind dabei die in Fig. 1 gezeigten Sensoren zu nennen, also der arterielle Drucksensor 28, der Dialysatoreingangsdrucksensor 30, der venöse Drucksensor 32, der arterielle Sicherheitsluftdetektor 24, der venöse Sicherheitsluftdetektor 18, der Drucksensor 50, etc.. Auf der anderen Seite steuert bzw. betätigt die Steuereinheit 60 Aktoren, welche in der extrakorporalen Blutbehandlungsvorrichtung 2 vorgesehen sind. Lediglich beispielhaft sind dabei die in Fig. 1 gezeigten Ventile, Pumpen, Schlauchklemmen, etc., also insbesondere das Dialysatoreinlassventil 42, das Dialysatorauslassventil 44, die Flusspumpe-Eingang 46, die Flusspumpe-Ausgang 48, die (arterielle) Blutpumpe 26, die arterielle Schlauchklemme 22, die venöse Schlauchklemme 20, etc. zu nennen.

Die Steuereinheit 60 steuert nach einem Ende der Blutbehandlungstherapie zunächst eine Reinfusion von Blut in den Patienten 15 derart, dass eine (Dialysier-) Flüssigkeit von dem Dialysierflüssigkeitskreislauf 8 über die Membran 10 des Dialysators 6 an den extrakorporalen Kreislauf 4 geliefert wird, welche bei der Reinfusion das in dem extrakorporalen Kreislauf 4 noch vorhandene Blut zu dem Patienten 15 hin verdrängt, um das Blut dem Patienten 15 sowohl über den venösen Abschnitt 14 als auch über den arteriellen Abschnitt 12 zurückzugeben. Dabei wird in vorteilhafter Weise ein Überdruck auf der Dialysierflüssigkeitsseite 11 aufgebaut, um die Flüssigkeit über die Membran 10 des Dialysators 6 in die Blutseite 9 zu drücken. Zusätzlich kann ein Unterdruck auf der Blutseite 9 aufgebaut werden, um die Flüssigkeit auch über die Membran 10 des Dialysators 6 in die Blutseite 9 zu ziehen.

Um die Reinfusion sowohl über den arteriellen Abschnitt 12 des extrakorporalen Kreislaufs 4 als auch über den venösen Abschnitt 14 des extrakorporalen Kreislaufs 4 abzubrechen (wenn das Blut vollständig reinfundiert wurde), schließt die Steuereinheit 46 die arterielle Schlauchklemme 22, sowie die venöse Schlauchklemme 20. Im Anschluss wird der Patient 15 von einem Pflegepersonal sowohl arteriell als auch venös abgekoppelt.

Fig. 2 zeigt die extrakorporale Blutbehandlungsvorrichtung (Dialysemaschine) 2 während des automatischen Entleerens des Dialysators 6. Es ist ersichtlich, dass nach dem Abkoppeln des Patienten 15 der arterielle Abschnitt 12 und der venöse Abschnitt 14 kurzgeschlossen bzw. fluidisch miteinander verbunden werden, beispielsweise über einen Verbinder/ Adapter 62. Der Dialysator 6 wurde - um das automatische Entleeren desselben zu ermöglichen - von einem Pflegepersonal gedreht, so dass sich in Fig. 2 der Dialysatorausgang 58 unten und der Dialysatoreingang 56 oben befindet. Der Dialysator 6 ist beim automatischen Entleeren desselben in vorteilhafter Weise vertikal ausgerichtet.

Die von der Steuereinheit 60 durchgeführte Steuerung des automatischen Entleerens des Dialysators 6 wird mit Bezug auf Fig. 2 beschrieben.

Bevorzugt ist die Steuereinheit 60 eingerichtet, zunächst einen Unterdruck auf der Dialysierflüssigkeitsseite 11 des Dialysators 6 zu erzeugen bzw. aufzubauen. Dazu steuert die Steuereinheit 60 die Flusspumpe-Ausgang 48 an, die in dem extrakorporalen Kreislauf 4 und in der Blutseite 9 des Dialysators 6 vorhandene Flüssigkeit in die Dialysierflüssigkeitsseite 11 des Dialysators 6 und aus dem Dialysator 6 heraus in den Dialysierflüssigkeitsabfluss 54 zu pumpen. Dabei ist die Flusspumpe-Eingang 46 bevorzugt gestoppt, das Dialysatoreinlassventil 42 geschlossen und das Dialysatorsauslassventil 44 geöffnet.

Weiterhin kann die Steuereinheit 60 die Kompressorpumpe 34 ansteuern, damit diese den Übertritt der Flüssigkeit von der Blutseite 9 über die Membran 10 des Dialysators 6 hin zu der Dialysierflüssigkeitsseite 11 unterstützt. Dazu drückt die Kompressorpumpe 34 bevorzugt Luft in den extrakorporalen Kreislauf 4. Beispielsweise wird das dritte Ventil 40 geöffnet, während das erste Ventil 36 und das zweite Ventil 38 geschlossen bleiben, und die Kompressorpumpe 34 pumpt Luft in die venöse Expansionskammer/ Luftfalle 16 (Steuerung/ Regelung der Luftzufuhr über den venösen Drucksensor 32). Durch dieses Einbringen von Luft in den extrakorporalen Kreislauf 4 wird ein Druck in dem extrakorporalen Kreislauf 4 aufgebaut und die in dem extrakorporalen Kreislauf 4 noch vorhandene Flüssigkeit wird zusätzlich auch über die Membran 10 des Dialysators 6 von der Blutseite 9 in die Dialysierflüssigkeitsseite 11 gedrückt.

Gemäß der vorliegenden Offenbarung ist eine Betätigung der Kompressorpumpe 34 beim automatischen Entleeren des Dialysators 6 optional, das heißt nicht zwingend erforderlich.

In jedem Fall ist die Steuereinheit 60 jedoch eingerichtet einen Transmembrandruck des Dialysators 6 auf einen konstanten, hohen Wert zu steuern bzw. zu regeln. Dabei hat sich insbesondere herausgestellt, dass der Transmembrandruck größer als 400 mmHg, insbesondere größer als 500 mmHg, beispielsweise 550 mmHg, sein sollte. Der Transmembrandruck kann von der Steuereinheit 60 lediglich über ein Ansteuern der Flusspumpe-Ausgang 48 (beispielsweise Ändern der Förderrate derselben) gesteuert/ geregelt werden. In anderen Worten kann grundsätzlich lediglich ein geeigneter Unterdruck auf der Dialysierflüssigkeitsseite 11 erzeugt werden. Alternativ kann durch ein zusätzliches Ansteuern der Kompressorpumpe 34 auch noch ein Überdruck auf der Blutseite 9 erzeugt werden.

Der Transmembrandruck wird von der Steuereinheit 60 in jedem Fall derart gesteuert bzw. geregelt, dass der maximale zulässige Transmembrandruck des verwendeten Dialysators 6, welcher beispielsweise vor einer Blutbehandlungstherapie von einem Anwender eingegeben bzw. eingelesen wird und der Blutbehandlungsvorrichtung 2, insbesondere der Steuereinheit 60 derselben somit bekannt ist, nicht überschritten wird. Insbesondere wird dadurch vermieden, dass Hohlfasern der Membran 10 reißen, und somit gegebenenfalls noch vorhandenen Blutpartikel in der Flüssigkeit in die Dialysierflüssigkeitsseite 11 übertreten können.

Wenn die Flüssigkeit aus dem extrakorporalen Kreislauf 4 vollständig von der Blutseite 9 über die Membran 10 des Dialysators 6 in die Dialysierflüssigkeitsseite 11 übergetreten ist und die Blutseite 9 des Dialysators 6 geleert ist, wird gemäß der vorliegenden Offenbarung der bereits anliegende Unterdruck auf der Dialysierflüssigkeitsseite 11, bzw. insbesondere der gesteuerte/ geregelte Transmembrandruck aufrechterhalten. Insbesondere hat sich offenbarungsgemäß herausgestellt, dass, wenn der Transmembrandruck auf einen derart hohen Wert gesteuert/ geregelt wird, sowohl Flüssigkeit als auch Luft über die Membran 10 des Dialysators 6 transportiert werden können. Spätestens an dieser Stelle muss der Dialysatorausgang 58 nach unten gerichtet/ unten sein. Durch den vorherrschenden Unterdruck und einen geleerten extrakorporalen Kreislauf 4 tritt nun Luft durch die Membran 10 des Dialysators 6 und verdrängt die noch in dem Dialysator 6 vorhandene Flüssigkeit auch aus der Dialysierflüssigkeitsseite 11 heraus hin zu dem Dialysierflüssigkeitsabfluss 54. Auch der Luftübertritt wird bevorzugt durch die Kompressorpumpe 34 unterstützt.

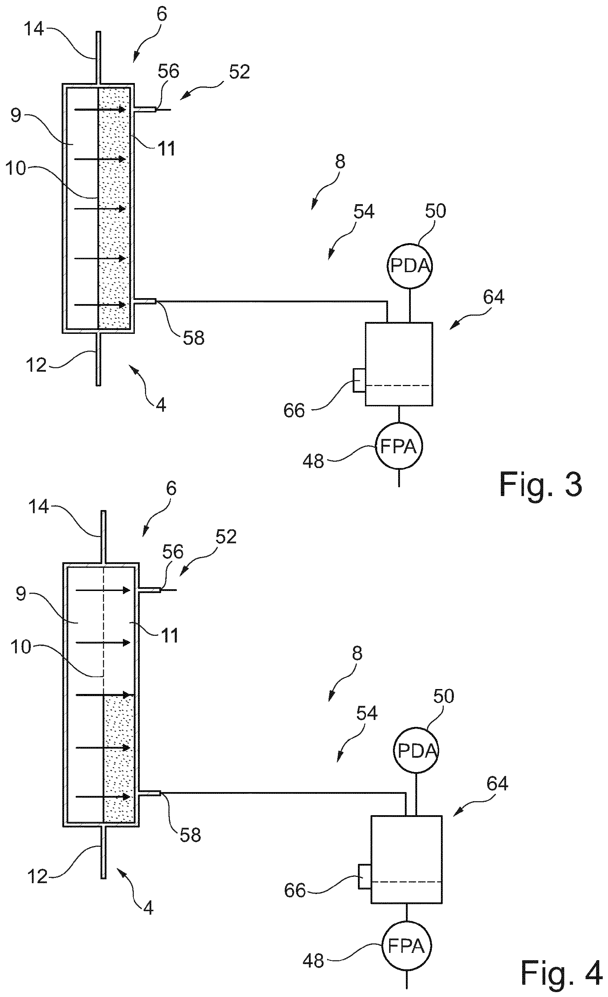

Fig. 3 zeigt eine Detailansicht des Dialysators 6 in einem Zustand, in welchem die Blutseite 9 desselben bereits vollständig entleert ist. Fig. 4 zeigt eine Detailansicht des Dialysators 6 in einem Zustand, in welchem die Dialysierflüssigkeitsseite 11 desselben gerade entleert wird. Wie insbesondere aus Fig. 4 hervorgeht, sammelt sich die übergetretene Luft zunächst in einem oberen Abschnitt des Dialysators 6 (im Bereich des/ nahe dem Dialysatoreingang/s 56) und verdrängt die Flüssigkeit nach unten zum Dialysatorausgang 58 hin.

Die Steuereinheit 60 ist bevorzugt eingerichtet, das automatische Entleeren des Dialysators 6 abzubrechen, wenn auch die Dialysierflüssigkeitsseite 11 des Dialysators 6 vollständig entleert ist. Bevorzugte Abbruchkriterien der vorliegenden Offenbarung werden zunächst unter Heranziehung von Fig. 3 und Fig. 4 beschrieben.

Grundsätzlich ist gemäß der vorliegenden Offenbarung ein zeitgesteuerter Abbruch des automatischen Entleerens denkbar. Beispielsweise kann die Steuereinheit 60 auf der Grundlage von ihr übermittelten Sensordaten erkennen, wann die Blutseite 9 des Dialysators 6 vollständig entleert ist. Wenn die Steuereinheit 60 weiß, wie lange für den verwendeten Dialysator 6 bei dem eingestellten Transmembrandruck eine Entleerung der Dialysierflüssigkeitsseite 11 gewöhnlich dauert, kann die Steuereinheit 60 das automatische Entleeren abbrechen, wenn eine entsprechende Zeitspanne verstrichen ist.

Besonders bevorzugt ist gemäß der vorliegenden Offenbarung ein sensorgesteuertes Abbrechen des automatischen Entleerens der Dialysierflüssigkeitsseite 11 des Dialysators 6. Dabei wertet die Steuereinheit 60 in vorteilhafter Weise ein Drucksignal bzw. einen Druckverlauf des in dem Dialysierflüssigkeitsabfluss 54 angeordneten Drucksensors 50 aus.

Alternativ kann das sensorgesteuerte Abbrechen des automatischen Entleerens auch über einen in dem Dialysierflüssigkeitskreislauf 8, insbesondere dem Dialysierflüssigkeitsabfluss 54, vorgesehenen Luftabscheider 64 erfolgen. Der Luftabscheider 64 dient während einer Blutbehandlungstherapie grundsätzlich einem Entfernen von Luft aus Dialysat und schützt die Flusspumpe-Ausgang 48 vor einem ungewollten Lufteintritt. In dem Luftabscheider 64 ist zumindest ein Flüssigkeitspegelsensor 66 vorgesehen. Wenn der Flüssigkeitspegelsensor 66 des Luftabscheiders 64 erfasst, dass der Flüssigkeitspegel in dem Luftabscheider 64 unter einen vorbestimmten Flüssigkeitspegel bzw. Pegelstand gefallen ist, bedeutet dies, dass Luft über den Dialysierflüssigkeitsabfluss 54 in den Luftabscheider 64 gelangt ist und somit auch die Dialysierflüssigkeitsseite 11 des Dialysators 6 geleert ist.

Da sich zwischen dem Dialysator 6 und dem Luftabscheider 64 in der Praxis ein relativ langer Schlauchabschnitt befindet (ca. 1 Meter), dauert es sehr lange, bis das automatische Entleeren abgebrochen wird, wenn der Luftabscheider 64 für das sensorgesteuerte Abbrechen verwendet wird. Vor diesem Hintergrund ist im Hinblick auf das sensorgesteuerte Abbrechen gemäß der vorliegenden Offenbarung grundsätzlich die Verwendung des Drucksensors (PDA) 50 bevorzugt. Denn eine Zeitdauer, nach welcher sich die Druckänderung durch den Schlauchabschnitt übertragen hat, ist wesentlich kürzer als eine Zeitdauer, nach welcher Luft über den Dialysierflüssigkeitsabfluss 54 in den Luftabscheider 64 gelangt ist. Auch wenn sowohl Fig. 3 als auch Fig. 4 im Wesentlichen schematische Ansichten sind, soll in beiden Figuren dennoch verdeutlicht werden, dass der Drucksensor 50 und der Luftabscheider 64 entfernt von dem Dialysator 6 angeordnet sind. Offenbarungsgemäß kann es auch vorgesehen sein, dass der Luftabscheider 64 und der Drucksensor 50 in einem Bauteil zusammengefasst sind, wie in Fig. 3 und Fig. 4 angedeutet ist.

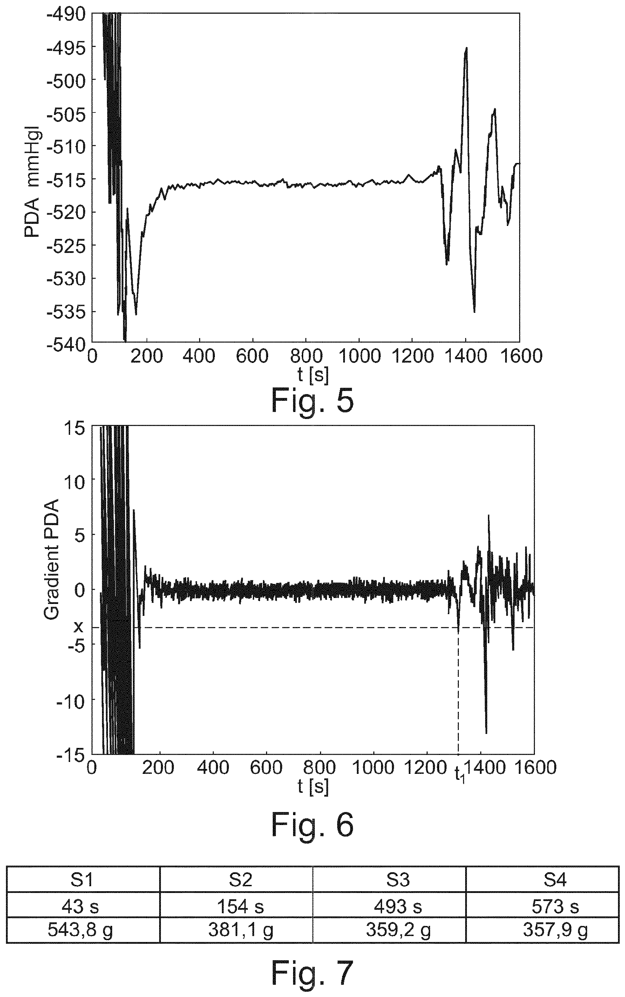

Das sensorgesteuerte Abbrechen des automatischen Entleerens der Dialysierflüssigkeitsseite 11 des Dialysators 6 wird noch genauer mit Bezug auf Fig. 5 und Fig. 6 beschrieben. Dabei zeigt Fig. 5 ein Diagramm, in welchem das Drucksignal bzw. der Druckverlauf des Drucksensors 50 über die Zeit dargestellt ist. Fig. 6 zeigt ein Diagramm, in welchem eine Steigung/ erste Ableitung/ ein Gradient des Drucksignals bzw. des Druckverlaufs des Drucksensors 50 über die Zeit dargestellt ist.

Die Steuereinheit 60 überwacht bevorzugt den von dem Drucksensor (PDA) 50 gemessenen Druck zum Ende des Leerungsprozesses hin, also beispielsweise ab etwa 200 Sekunden in Fig. 5 bzw. Fig. 6. Wenn der Dialysator 6 vollständig geleert ist, tritt Luft in den Dialysierflüssigkeitsabfluss 54 über. Dies geht mit einem Absinken des Drucks in dem Dialysierflüssigkeitsabfluss 54 einher, wobei das Absinken von dem Drucksensor (PDA) 50 gemessen wird. Aus Fig. 5 und Fig. 6 geht hervor, dass der von dem Drucksensor 50 gemessene Druck während des Entleerens nahezu konstant ist. Dasselbe gilt entsprechend auch für die Steigung des Drucksignals. Nach ca. 1200 Sekunden fängt das Signal in Fig. 5 an zu schwanken. Dies ist ein Indiz dafür, dass nun (auch) Luft an dem Drucksensor 50 anliegt. Ebenso verändert sich die Steigung des Drucksignals (siehe Fig. 6). Gemäß der vorliegenden Offenbarung hat sich herausgestellt, dass das Abbrechen des automatischen Entleerens des Dialysators 6 am besten anhand der Steigung bzw. ersten Ableitung des Drucksignals vorgenommen wird. Wenn die Steigung einen bestimmten negativen vorbestimmten Wert x, welcher bevorzugt zwischen -3 und -5, vorzugsweise auf (etwa) -4 eingestellt wird, unterschreitet, ist das Abbruchkriterium erfüllt. Dies ist in Fig. 6 zu Zeitpunkt t1 der Fall.

Fig. 7 zeigt abschließend eine Tabelle, in welcher für verschiedene Dialysator-Entleerungsverfahren eine erforderliche Zeit und ein verbleibendes Entsorgungsgewicht angegeben ist. Dabei wurde für sämtliche Versuche derselbe Dialysator ("Xevonta Hi23") verwendet.

In dem mit "S1" bezeichneten Versuch wurde zunächst der extrakorporale Kreislauf bzw. das Blutschlauchsystem von dem Dialysator abgekoppelt und in einen Beutel oder in einen Waste-Port der Blutbehandlungsvorrichtung entleert. Im Anschluss wurde ein Dialysierflüssigkeitszufluss von dem Dialysator abgekoppelt und wurde im Dialysator noch vorhandene Flüssigkeit zumindest zum Teil über den Dialysierflüssigkeitsabfluss abgesaugt. Es hat sich gezeigt, dass dieses Entleerungsverfahren lediglich 43 Sekunden dauert. Das Entsorgungsgewicht betrug 543,8 Gramm.

In dem mit "S2" bezeichneten Versuch wurde die Dialysator-Entleerung wie im Stand der Technik der EP 1 996 253 B1, also über ein in dem Dialysierflüssigkeitszufluss vorgesehenes Ventil vorgenommen. Es hat sich gezeigt, dass dieses Entleerungsverfahren 154 Sekunden dauert. Das Entsorgungsgewicht betrug 381,1 Gramm.

In dem mit "S3" bezeichneten Versuch wurde die Dialysator-Entleerung gemäß der vorliegenden Offenbarung vorgenommen, wobei ein sensorgesteuertes Abbrechen des automatischen Entleerens des Dialysators über den Drucksensor im Dialysierflüssigkeitsabfluss vorgenommen wurde. Es hat sich gezeigt, dass dieses Entleerungsverfahren 493 Sekunden dauert. Das Entsorgungsgewicht konnte bis auf 359,2 Gramm reduziert werden.

In dem mit "S4" bezeichneten Versuch wurde die Dialysator-Entleerung gemäß der vorliegenden Offenbarung vorgenommen, wobei ein sensorgesteuertes Abbrechen des automatischen Entleerens des Dialysators über den Luftabscheider im Dialysierflüssigkeitsabfluss vorgenommen wurde. Es hat sich gezeigt, dass dieses Entleerungsverfahren 573 Sekunden dauert. Das Entsorgungsgewicht konnte bis auf 357,9 Gramm reduziert werden.

Es hat sich somit gezeigt, dass gemäß der vorliegenden Offenbarung (vgl. Versuche "S3" und "S4") eine Reduzierung des Entsorgungsgewichts gegenüber dem Stand der Technik möglich ist (gilt insbesondere im Hinblick auf Versuch "S1", jedoch auch im Hinblick auf Versuch "S2"). Zwar dauert das Leeren des Dialysators gemäß der vorliegenden Offenbarung lange, jedoch wird dieser Nachteil vor dem Hintergrund der erzielbaren Ersparnisse bei den Entsorgungskosten gerne in Kauf genommen. Darüber hinaus gilt, dass die längere Zeitdauer in der Praxis keine wesentliche Rolle spielt. Denn die Dialysator-Entleerung findet automatisch (d.h. ohne Eingriff des Pflegepersonals) statt, und es wird letztendlich lediglich die Zeit nach einem Abkoppeln eines Patienten genutzt, insbesondere bis dieser seinen Behandlungsplatz verlassen hat.

### Bezugszeichenliste

- 2: extrakorporale Blutbehandlungsvorrichtung/ Dialysemaschine
- 4: extrakorporaler Kreislauf
- 6: Dialysator
- 8: Dialysierflüssigkeitskreislauf
- 9: Blutseite
- 10: Membran
- 11: Dialysierflüssigkeitsseite
- 12: arterieller Abschnitt
- 14: venöser Abschnitt
- 15: Patient
- 16: venöse Expansionskammer/ Luftfalle
- 18: venöser Sicherheitsluftdetektor
- 20: venöse Schlauchklemme
- 22: arterielle Schlauchklemme
- 24: arterieller Sicherheitsluftdetektor
- 26: Blutpumpe
- 28: arterieller Drucksensor
- 30: Dialysatoreingangsdrucksensor
- 32: venöser Drucksensor
- 34: Kompressorpumpe
- 36: erstes Ventil
- 38: zweites Ventil
- 40: drittes Ventil
- 42: Dialysatoreinlassventil
- 44: Dialysatorauslassventil
- 46: Flusspumpe-Eingang
- 48: Flusspumpe-Ausgang
- 50: Drucksensor
- 52: Dialysierflüssigkeitszufluss
- 54: Dialysierflüssigkeitsabfluss
- 56: Dialysatoreingang
- 58: Dialysatorausgang
- 60: Steuereinheit
- 62: Verbinder/ Adapter
- 64: Luftabscheider
- 66: Flüssigkeitspegelsensor

## Patentansprüche

1. Extrakorporale Blutbehandlungsvorrichtung (2) zur Verwendung in einer Blutbehandlungstherapie, mit:
einem extrakorporalen Kreislauf (4);
einem Dialysierflüssigkeitskreislauf (8); und
einem Dialysator (6) umfassend eine Blutseite (9), welche mit dem extrakorporalen Kreislauf (4) fluidisch verbunden ist, und eine Dialysierflüssigkeitsseite (11), welche mit dem Dialysierflüssigkeitskreislauf (8) fluidisch verbunden ist, wobei
die Blutseite (9) des Dialysators (6) und die Dialysierflüssigkeitsseite (11) des Dialysators (6) über eine in dem Dialysator (6) vorgesehene Membran (10) voneinander getrennt sind, und
die extrakorporale Blutbehandlungsvorrichtung (2) ferner eine Steuereinheit (60) enthält, welche zum automatischen Entleeren des Dialysators (6) nach einem Ende der Blutbehandlungstherapie durch Einstellen eines negativen Drucks bzw. Unterdrucks in dem Dialysierflüssigkeitskreislauf (8) und einem damit einhergehenden Übertritt einer Flüssigkeit von der Blutseite (9) über die Membran (10) des Dialysators (6) in die Dialysierflüssigkeitsseite (11) eingerichtet ist,
**dadurch gekennzeichnet, dass**
die Steuereinheit (60), wenn die Flüssigkeit aus dem extrakorporalen Kreislauf (4) vollständig von der Blutseite (9) über die Membran (10) des Dialysators (6) in die Dialysierflüssigkeitsseite (11) übergetreten ist und die Blutseite (9) des Dialysators (6) geleert ist, eingerichtet ist, durch ein fortgesetztes Einstellen des negativen Drucks bzw. Unterdrucks in dem Dialysierflüssigkeitskreislauf (8) einen Übertritt von Luft von der Blutseite (9) über die Membran (10) des Dialysators (6) zu der Dialysierflüssigkeitsseite (11) und ein Verdrängen der Flüssigkeit aus der Dialysierflüssigkeitsseite (11) des Dialysators (6) heraus hin zu einem Dialysierflüssigkeitsabfluss (54) zu bewirken, um auch die Dialysierflüssigkeitsseite (11) des Dialysators (6) automatisch zu entleeren.

2. Extrakorporale Blutbehandlungsvorrichtung (2) nach Anspruch 1, **dadurch gekennzeichnet, dass** der Dialysator (6) beim automatischen Entleeren desselben derart an der extrakorporalen Blutbehandlungsvorrichtung (2) angeordnet bzw. ausgerichtet ist, dass sich ein Dialysierflüssigkeitsausgang (58), welcher mit dem Dialysierflüssigkeitsabfluss (54) verbunden ist, unter einem Dialysierflüssigkeitseingang (56), welcher mit einem Dialysierflüssigkeitszufluss (52) verbunden ist, angeordnet ist.

3. Extrakorporale Blutbehandlungsvorrichtung (2) nach Anspruch 1 oder 2, **dadurch gekennzeichnet, dass** die Steuereinheit (60) eingerichtet ist, den negativen Druck bzw. Unterdruck in dem Dialysierflüssigkeitskreislauf (8) derart zu erzeugen, dass eine Flusspumpe-Ausgang (48), welche eine Fluidpumpe in dem Dialysierflüssigkeitsabfluss (54) des Dialysierflüssigkeitskreislaufs (8) ist, angesteuert wird, die Flüssigkeit oder die Luft aus dem Dialysator (6) heraus in den Dialysierflüssigkeitsabfluss (54) zu pumpen.

4. Extrakorporale Blutbehandlungsvorrichtung (2) nach einem der vorhergehenden Ansprüche, **dadurch gekennzeichnet, dass** die Steuereinheit (60) eingerichtet ist, eine in dem extrakorporalen Kreislauf (4) vorgesehene Kompressorpumpe (34) anzusteuern bzw. zu betätigen, damit diese den Übertritt der Flüssigkeit oder der Luft von der Blutseite (9) über die Membran (10) des Dialysators (6) hin zu der Dialysierflüssigkeitsseite (11) unterstützt.

5. Extrakorporale Blutbehandlungsvorrichtung (2) nach einem der vorhergehenden Ansprüche, **dadurch gekennzeichnet, dass** die Steuereinheit (60) eingerichtet ist, beim automatischen Entleeren des Dialysators (6) einen Transmembrandruck des Dialysators (6) auf einen Druck zu steuern oder zu regeln, welcher größer einem vorbestimmten Wert und kleiner einem dialysatorspezifischen, maximal zulässigen Transmembrandruck ist.

6. Extrakorporale Blutbehandlungsvorrichtung (2) nach Anspruch 5, **dadurch gekennzeichnet, dass** die Steuereinheit (60) eingerichtet ist, den Transmembrandruck des Dialysators (6) auf einen Druck zu steuern oder regeln, welcher größer als 400 mmHg, bevorzugt größer als 500 mmHg ist.

7. Extrakorporale Blutbehandlungsvorrichtung (2) nach einem der vorhergehenden Ansprüche, **dadurch gekennzeichnet, dass** die Steuereinheit (60) eingerichtet ist, das automatische Entleeren der Dialysierflüssigkeitsseite (11) des Dialysators (6) sensorgesteuert abzubrechen.

8. Extrakorporale Blutbehandlungsvorrichtung (2) nach Anspruch 7, **dadurch gekennzeichnet, dass** die Steuereinheit (60) eingerichtet ist, ein Drucksignal bzw. einen Druckverlauf eines in dem Dialysierflüssigkeitsabfluss (54) angeordneten Drucksensors (50), welcher einen Druck in dem Dialysierflüssigkeitsabfluss (54) misst bzw. überwacht, auszuwerten, und das automatische Entleeren der Dialysierflüssigkeitsseite (11) auf der Grundlage des Drucksignals bzw. des Druckverlaufs des Drucksensors (50) abzubrechen.

9. Extrakorporale Blutbehandlungsvorrichtung (2) nach Anspruch 8, **dadurch gekennzeichnet, dass** die Steuereinheit (60) eingerichtet ist, eine Steigung bzw. eine erste Ableitung des Drucksignals bzw. des Druckverlaufs des Drucksensors (50) auszuwerten, und das automatische Entleeren der Dialysierflüssigkeitsseite (11) abzubrechen, wenn die Steigung bzw. erste Ableitung des Drucksignals bzw. des Druckverlaufs einen vorbestimmten ersten Grenzwert unterschreitet.

10. Extrakorporale Blutbehandlungsvorrichtung (2) nach Anspruch 7, **dadurch gekennzeichnet, dass** die Steuereinheit (60) eingerichtet ist, das automatische Entleeren der Dialysierflüssigkeitsseite (11) sensorgesteuert abzubrechen, wenn von einem in dem Dialysierflüssigkeitskreislauf (8) vorgesehenen bzw. angeordneten Luftabscheider (64) erfasst bzw. gemessen wird, dass ein Flüssigkeitspegel des Luftabscheiders (64) unter einen vorbestimmten Flüssigkeitspegel bzw. Pegelstand gefallen ist.

11. Extrakorporale Blutbehandlungsvorrichtung (2) nach einem der vorhergehenden Ansprüche, **dadurch gekennzeichnet, dass** die Steuereinheit (60) eingerichtet ist, das automatische Entleeren der Dialysierflüssigkeitsseite (11) des Dialysators (6) zeitgesteuert abzubrechen.

12. Verfahren zum automatischen Entleeren eines Dialysators (6) nach einem Ende einer Blutbehandlungstherapie, insbesondere durchgeführt oder durchzuführen in einer extrakorporalen Blutbehandlungsvorrichtung (2) gemäß einem der vorhergehenden Ansprüche, mit den Schritten:
Einstellen eines negativen Drucks bzw. Unterdrucks in einem Dialysierflüssigkeitskreislauf (8) und damit einhergehendes Übertreten einer Flüssigkeit von einer Blutseite (9) des Dialysators (6) über eine Membran (10) des Dialysators (6) in eine Dialysierflüssigkeitsseite (11) des Dialysators (6), und
wenn die Flüssigkeit aus einem extrakorporalen Kreislauf (4) vollständig von der Blutseite (9) über die Membran (10) des Dialysators (6) in die Dialysierflüssigkeitsseite (11) übergetreten ist und die Blutseite (9) des Dialysators (6) geleert ist, Fortgesetztes Einstellen des negativen Drucks bzw. Unterdrucks in dem Dialysierflüssigkeitskreislauf (8) zum Bewirken eines Übertritts von Luft von der Blutseite (9) über die Membran (10) des Dialysators (6) zu der Dialysierflüssigkeitsseite (11) und eines Verdrängens der Flüssigkeit aus der Dialysierflüssigkeitsseite (11) des Dialysators (6) heraus hin zu einem Dialysierflüssigkeitsabfluss (54), um auch die Dialysierflüssigkeitsseite (11) des Dialysators (6) automatisch zu entleeren.

## Claims

1. An extracorporeal blood treatment device (2) for use in a blood treatment therapy, comprising:
an extracorporeal circuit (4);
a dialysis liquid circuit (8); and
a dialyzer (6) comprising a blood side (9) fluidically connected to the extracorporeal circuit (4) and a dialysis liquid side (11) fluidically connected to the dialysis liquid circuit (8), wherein
the blood side (9) of the dialyzer (6) and the dialysis liquid side (11) of the dialyzer (6) are separated from each other via a membrane (10) provided in the dialyzer (6), and
the extracorporeal blood treatment device (2) further comprises a control unit (60) configured to automatically empty the dialyzer (6) after an end of the blood treatment therapy by setting a negative pressure in the dialysis liquid circuit (8) and a concomitant transfer of liquid from the blood side (9) via the membrane (10) of the dialyzer (6) into the dialysis liquid side (11),
**characterized in that**
the control unit (60) is configured, when the liquid from the extracorporeal circuit (4) has completely transferred from the blood side (9) via the membrane (10) of the dialyzer (6) into the dialysis liquid side (11) and the blood side (9) of the dialyzer (6) has been emptied, to cause, by a continued setting of the negative pressure in the dialysis liquid circuit (8), a transfer of air from the blood side (9) via the membrane (10) of the dialyzer (6) to the dialysis liquid side (11) and a displacement of the liquid out of the dialysis liquid side (11) of the dialyzer (6) to a dialysis liquid outflow (54), in order to also automatically empty the dialysis liquid side (11) of the dialyzer (6).

2. The extracorporeal blood treatment device (2) according to claim 1, **characterized in that** the dialyzer (6) is arranged or oriented during automatic emptying thereof on the extracorporeal blood treatment device (2) such that a dialysis liquid exit (58) connected to the dialysis liquid outflow (54) is arranged below a dialysis liquid inlet (56) connected to a dialysis liquid inflow (52).

3. The extracorporeal blood treatment device (2) according to claim 1 or 2, **characterized in that** the control unit (60) is configured to generate the negative pressure in the dialysis liquid circuit (8) such that a flux-pump outlet (48), which is a fluid pump in the dialysis liquid outflow (54) of the dialysis liquid circuit (8), is driven to pump the liquid or air out of the dialyzer (6) into the dialysis liquid outflow (54).

4. The extracorporeal blood treatment device (2) according to one of the preceding claims, **characterized in that** the control unit (60) is configured to drive or actuate a compressor pump (34) provided in the extracorporeal circuit (4) to support the transfer of the liquid or air from the blood side (9) via the membrane (10) of the dialyzer (6) to the dialysis liquid side (11).

5. The extracorporeal blood treatment device (2) according to one of the preceding claims, **characterized in that** the control unit (60) is configured to control or regulate a transmembrane pressure of the dialyzer (6) during automatic emptying of the dialyzer (6) to a pressure that is greater than a predetermined value and smaller than a dialyzer-specific, maximum permissible transmembrane pressure.

6. The extracorporeal blood treatment device (2) according to claim 5, **characterized in that** the control unit (60) is configured to control or regulate the transmembrane pressure of the dialyzer (6) to a pressure greater than 400 mmHg, preferably greater than 500 mmHg.

7. The extracorporeal blood treatment device (2) according to one of the preceding claims, **characterized in that** the control unit (60) is configured to stop the automatic emptying of the dialysis liquid side (11) of the dialyzer (6) in a sensor-controlled manner.

8. The extracorporeal blood treatment device (2) according to claim 7, **characterized in that** the control unit (60) is configured to evaluate a pressure signal or pressure course of a pressure sensor (50) arranged in the dialysis liquid outflow (54), which measures or monitors a pressure in the dialysis liquid outflow (54), and to stop the automatic emptying of the dialysis liquid side (11) based on the pressure signal or pressure course of the pressure sensor (50).

9. The extracorporeal blood treatment device (2) according to claim 8, **characterized in that** the control unit (60) is configured to evaluate a slope or a first derivative of the pressure signal or of the pressure course of the pressure sensor (50), and to stop the automatic emptying of the dialysis liquid side (11) if the slope or first derivative of the pressure signal or the pressure course falls below a predetermined first limit value.

10. The extracorporeal blood treatment device (2) according to claim 7, **characterized in that** the control unit (60) is configured to stop the automatic emptying of the dialysis liquid side (11) in a sensor-controlled manner if an air separator (64) provided or arranged in the dialysis liquid circuit (8) detects or measures that a liquid level of the air separator (64) has fallen below a predetermined liquid level or gauge height.

11. The extracorporeal blood treatment device (2) according to one of the preceding claims, **characterized in that** the control unit (60) is configured to stop the automatic emptying of the dialysis liquid side (11) of the dialyzer (6) in a time-controlled manner.

12. A method for automatically emptying a dialyzer (6) after an end of a blood treatment therapy, in particular performed or to be performed in an extracorporeal blood treatment device (2) according to one of the preceding claims, comprising the steps of:
setting a negative pressure in a dialysis liquid circuit (8) and concomitant transfer of a liquid from a blood side (9) of the dialyzer (6) via a membrane (10) of the dialyzer (6) into a dialysis liquid side (11) of the dialyzer (6), and
when the liquid from an extracorporeal circuit (4) has completely passed from the blood side (9) via the membrane (10) of the dialyzer (6) into the dialysis liquid side (11) and the blood side (9) of the dialyzer (6) has been emptied, continuing to set the negative pressure in the dialysis liquid circuit (8) to cause a transfer of air from the blood side (9) via the membrane (10) of the dialyzer (6) to the dialysis liquid side (11) and a displacement of liquid out of the dialysis liquid side (11) of the dialyzer (6) to a dialysis liquid outflow (54) in order to also automatically empty the dialysis liquid side (11) of the dialyzer (6).

## Revendications

1. Dispositif de traitement sanguin extracorporel (2) destiné à être utilisé dans une thérapie de traitement sanguin, avec :
un circuit extracorporel (4) ;
un circuit de liquide de dialyse (8) ; et
un dialyseur (6) comprenant un côté sang (9), lequel est connecté par voie fluidique au circuit extracorporel (4) et un côté liquide de dialyse (11), lequel est connecté par voie fluidique au circuit de liquide de dialyse (8), dans lequel
le côté sang (9) du dialyseur (6) et le côté liquide de dialyse (11) du dialyseur (6) sont séparés l'un de l'autre par une membrane (10) prévue dans le dialyseur (6), et
le dispositif de traitement sanguin extracorporel (2) contient en outre une unité de commande (60), laquelle est configurée pour vider automatiquement le dialyseur (6) après une fin de la thérapie de traitement sanguin en établissant une pression négative ou une dépression dans le circuit de liquide de dialyse (8) et en provoquant ainsi le passage concomitant d'un liquide du côté sang (9) au côté liquide de dialyse (11) par la membrane (10) du dialyseur (6),
**caractérisé en ce que**
l'unité de commande (60) est configurée pour, lorsque le liquide provenant du circuit extracorporel (4) est complètement passé du côté sang (9) par la membrane (10) du dialyseur (6) au côté liquide de dialyse (11) et que le côté sang (9) du dialyseur (6) est vidé, par un réglage poursuivi de la pression négative ou de la dépression dans le circuit de liquide de dialyse (8), faire passer de l'air du côté sang (9) par la membrane (10) du dialyseur (6) au côté liquide de dialyse (11) et provoquer une expulsion du liquide du côté liquide de dialyse (11) du dialyseur (6) vers un écoulement de liquide de dialyse (54) pour également vider automatiquement le côté liquide de dialyse (11) du dialyseur (6).

2. Dispositif de traitement sanguin extracorporel (2) selon la revendication 1, **caractérisé en ce que,** lors de son vidage automatique, le dialyseur (6) est disposé ou aligné sur le dispositif de traitement sanguin extracorporel (2) de sorte qu'une sortie de liquide de dialyse (58) connectée à l'écoulement de liquide de dialyse (54) est disposée sous une entrée de liquide de dialyse (56), laquelle est connectée à un afflux de liquide de dialyse (52).

3. Dispositif de traitement sanguin extracorporel (2) selon la revendication 1 ou 2, **caractérisé en ce que** l'unité de commande (60) est configurée pour générer la pression négative ou la dépression dans le circuit de liquide de dialyse (8) de sorte qu'une sortie de pompe à flux (48), laquelle est une pompe à fluide dans l'écoulement de liquide de dialyse (54) du circuit de liquide de dialyse (8), est commandée pour pomper le liquide ou l'air hors du dialyseur (6) dans l'écoulement de liquide de dialyse (54).

4. Dispositif de traitement sanguin extracorporel (2) selon l'une quelconque des revendications précédentes, **caractérisé en ce que** l'unité de commande (60) est configurée pour commander ou actionner une pompe à compresseur (34) prévue dans le circuit extracorporel (4) pour que celle-ci soutienne le passage du liquide ou de l'air du côté sang (9) au côté liquide de dialyse (11) par la membrane (10) du dialyseur (6).

5. Dispositif de traitement sanguin extracorporel (2) selon l'une quelconque des revendications précédentes, **caractérisé en ce que** l'unité de commande (60) est configurée pour commander ou réguler, lors du vidage automatique du dialyseur (6), une pression transmembranaire du dialyseur (6) à une pression, laquelle est supérieure à une valeur prédéterminée et inférieure à une pression transmembranaire maximale admissible spécifique au dialyseur.

6. Dispositif de traitement sanguin extracorporel (2) selon la revendication 5, **caractérisé en ce que** l'unité de commande (60) est configurée pour commander ou réguler la pression transmembranaire du dialyseur (6) à une pression, laquelle est supérieure à 400 mmHg, de préférence supérieure à 500 mmHg.

7. Dispositif de traitement sanguin extracorporel (2) selon l'une quelconque des revendications précédentes, **caractérisé en ce que** l'unité de commande (60) est configurée pour interrompre par capteur le vidage automatique du côté liquide de dialyse (11) du dialyseur (6).

8. Dispositif de traitement sanguin extracorporel (2) selon la revendication 7, **caractérisé en ce que** l'unité de commande (60) est configurée pour évaluer un signal de pression ou une courbe de pression d'un capteur de pression (50) disposé dans l'écoulement de liquide de dialyse (54), lequel mesure ou surveille une pression dans l'écoulement de liquide de dialyse (54), et pour interrompre le vidage automatique du côté liquide de dialyse (11) sur la base du signal de pression ou de la courbe de pression du capteur de pression (50).

9. Dispositif de traitement sanguin extracorporel (2) selon la revendication 8, **caractérisé en ce que** l'unité de commande (60) est configurée pour évaluer une pente ou une première dérivée du signal de pression ou de la courbe de pression du capteur de pression (50) et interrompre le vidage automatique du côté liquide de dialyse (11) lorsque la pente ou la première dérivée du signal de pression ou de la courbe de pression passe sous une première valeur limite prédéterminée.

10. Dispositif de traitement sanguin extracorporel (2) selon la revendication 7, **caractérisé en ce que** l'unité de commande (60) est configurée pour interrompre par capteur le vidage automatique du côté liquide de dialyse (11) lorsqu'un séparateur d'air (64) prévu ou disposé dans le circuit de liquide de dialyse (8) détecte ou mesure qu'un niveau de liquide du séparateur d'air (64) est tombé sous un niveau de liquide prédéterminé.

11. Dispositif de traitement sanguin extracorporel (2) selon l'une quelconque des revendications précédentes, **caractérisé en ce que** l'unité de commande (60) est configurée pour interrompre en fonction du temps le vidage automatique du côté liquide de dialyse (11) du dialyseur (6).

12. Procédé de vidage automatique d'un dialyseur (6) après une fin d'une thérapie de traitement sanguin, en particulier réalisé ou à réaliser dans un dispositif de traitement sanguin extracorporel (2) selon l'une quelconque des revendications précédentes, avec les étapes de :
réglage d'une pression négative ou dépression dans un circuit de liquide de dialyse (8) et passage concomitant d'un liquide d'un côté sang (9) du dialyseur (6) par une membrane (10) du dialyseur (6) à un côté liquide de dialyse (11) du dialyseur (6), et
lorsque le liquide provenant du circuit extracorporel (4) est complètement passé du côté sang (9) par la membrane (10) du dialyseur (6) au côté liquide de dialyse (11) et que le côté sang (9) du dialyseur (6) est vidé, poursuite du réglage de la pression négative ou de la dépression dans le circuit de liquide de dialyse (8) pour provoquer un passage d'air du côté sang (9) par la membrane (10) du dialyseur (6) au côté liquide de dialyse (11) et expulser le liquide du côté liquide de dialyse (11) du dialyseur (6) vers un écoulement de liquide de dialyse (54) pour également vider automatiquement le côté liquide de dialyse (11) du dialyseur (6).
